# EUROPÄISCHE PATENTANMELDUNG

(11) **EP 0 654 468 A1**
(43) Veröffentlichungstag der Anmeldung: **24.05.1995**
(21) Anmeldenummer: 94117746.1
(22) Anmeldetag: 10.11.1994
(51) Int. Cl.: C07D 249/12, A01N 47/38

(54) **Substituierte Carbamoyltriazole, ihre Verwendung als Herbizide, sowie Triazolzwischenprodukte**

(30) Priorität: 23.11.1993 DE 4339863
(71) Anmelder: BAYER AG, 51368 Leverkusen (DE)
(72) Erfinder: Andree, Dr. Roland, D-40764 Langenfeld (DE); Dollinger, Dr. Markus, D-51381 Leverkusen (DE); Santel, Dr. Hans-Joachim, D-51371 Leverkusen (DE)

(57) **Zusammenfassung**

Die Erfindung betrifft neue substituierte Carbamoyltriazole der allgemeinen Formel (I) in welcher 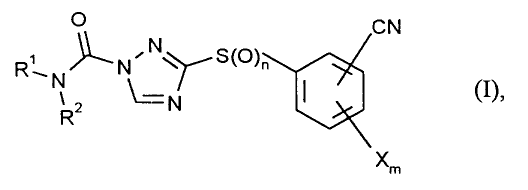
- m: für die Zahlen 0 bis 4 steht,
- n: für die Zahlen 0, 1 oder 2 steht,
- R¹: für einen jeweils gegebenenfalls substituierten Rest der Reihe Alkyl, Alkenyl oder Alkinyl steht,
- R²: für einen jeweils gegebenenfalls substituierten Rest der Reihe Alkyl, Alkenyl oder Alkinyl steht oder zusammen mit R¹ für Alkandiyl steht und
- X: für Halogen, Hydroxy, Amino, Mercapto oder für einen jeweils gegebenenfalls substituierten Rest der Reihe Alkyl, Alkoxy, Alkylthio, Alkylamino, Alkanoylamino, Alkylsulfonylamino, Cycloalkyloxy, Cycloalkylthio, Cycloalkylamino, Cycloalkylalkoxy, Cycloalkylalkylthio, Cycloalkylalkylamino, Aryloxy, Arylthio, Arylamino, Arylcarbonyl, Arylsulfonyl, Arylalkyl, Arylalkoxy, Arylalkylthio, Arylalkylamino, Arylalkylcarbonyl oder Arylalkylsulfonyl steht,
ein Verfahren zur ihrer Herstellung und ihre Verwendung als Herbizide, sowie entsprechende N - unsubstituierte Triazolzwischenprodukte.

## Beschreibung

Die Erfindung betrifft neue substituierte Carbamoyltriazole, ein Verfahren zu ihrer Herstellung und ihre Verwendung als Herbizide, sowie neue Zwischenprodukte.

Es ist bereits bekannt, daß bestimmte substituierte Carbamoyltriazole, wie z.B. die Verbindung 1-(Diethylaminocarbonyl)-3-(2,4,6-trimethylphenylsulfonyl)-1,2,4-triazol, herbizide Eigenschaften aufweisen (vgl. EP-A332133).

Die herbizide Wirksamkeit bzw. die Verträglichkeit dieser bekannten Verbindungen gegenüber Kulturpflanzen ist jedoch nicht immer ganz zufriedenstellend.

Es wurden nun die neuen substituierten Carbamoyltriazole der allgemeinen Formel (I) gefunden, 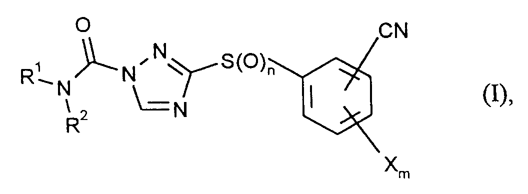
in welcher
- m: für die Zahlen 0 bis 4 steht,
- n: für die Zahlen 0, 1 oder 2 steht,
- R¹: für einen jeweils gegebenenfalls substituierten Rest der Reihe Alkyl, Alkenyl oder Alkinyl steht,
- R²: für einen jeweils gegebenenfalls substituierten Rest der Reihe Alkyl, Alkenyl oder Alkinyl steht oder zusammen mit R¹ für Alkandiyl steht und
- X: für Halogen, Hydroxy, Amino, Mercapto oder für einen jeweils gegebenenfalls substituierten Rest der Reihe Alkyl, Alkoxy, Alkylthio, Alkylamino, Alkanoylamino, Alkylsulfonylamino, Cycloalkyloxy, Cycloalkylthio, Cycloalkylamino, Cycloalkylalkoxy, Cycloalkylalkylthio, Cycloalkylalkylamino, Aryloxy, Arylthio, Arylamino, Arylcarbonyl, Arylsulfonyl, Arylalkyl, Arylalkoxy, Arylalkylthio, Arylalkylamino, Arylalkylcarbonyl oder Arylalkylsulfonyl steht.

Man erhält die neuen substituierten Carbamoyltriazole der allgemeinen Formel (I), wenn man Carbamidsäurechloride der allgemeinen Formel (II) 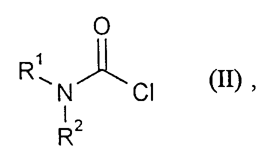
in welcher
R¹ und R²die oben angegebenen Bedeutungen haben,
mit substituierten Triazolen der allgemeinen Formel (III) 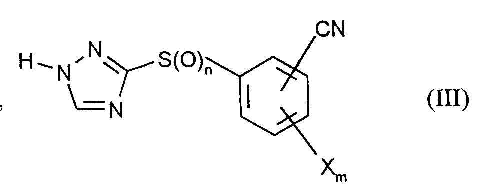
in welcher
m, n und X die oben angegebenen Bedeutungen haben,
gegebenenfalls in Gegenwart eines Säureakzeptors und gegebenenfalls in Gegenwart eines Verdünnungsmittels umsetzt,
gegebenenfalls die so erhaltenen Verbindungen der Formel (I), in welcher n für 0 steht, durch Umsetzung mit einem Oxidationsmittel in entsprechende Verbindungen der Formel (I), in welcher n für 1 oder 2 steht umwandelt und/oder gegebenenfalls die so erhaltenen Verbindungen der Formel (I), in welcher X für Halogen steht, durch Umsetzung mit Verbindungen der Formel (IV)

MX¹ (IV),

in welcher
- M: für Wasserstoff oder ein Metalläquivalent steht und
- X¹: mit Ausnahme von Halogen und Alkyl die oben für X angegebene Bedeutung hat,
in entsprechend derivatisierte Verbindungen der Formel (I) umwandelt.

Die neuen substituierten Carbamoyltriazole der allgemeinen Formel (I) zeichnen sich durch starke und selektive herbizide Wirksamkeit aus.

Überraschenderweise zeigen die erfindungsgemäßen Verbindungen der Formel (I) erheblich stärkere und besser selektive herbizide Wirkung, als die aus dem Stand der Technik bekannten Verbindungen ähnlicher Struktur und gleicher Wirkungsrichtung, wie z.B. die Verbindung 1-(Diethylaminocarbonyl)-3-(2,4,6-trimethylphenylsulfonyl)-1,2,4-triazol.

Gegenstand der Erfindung sind vorzugsweise die Verbindungen der Formel (I), in welcher
- m: für die Zahlen 0, 1, 2, 3 oder 4 steht,
- n: für die Zahlen 0, 1 oder 2 steht,
- R¹: für einen jeweils gegebenenfalls durch Halogen substituierten Rest der Reihe Alkyl, Alkenyl oder Alkinyl mit jeweils bis zu 6 Kohlenstoffatomen steht,
- R²: für einen jeweils gegebenenfalls durch Halogen substituierten Rest der Reihe Alkyl, Alkenyl oder Alkinyl mit jeweils bis zu 6 Kohlenstoffatomen steht oder zusammen mit R¹ für Alkandiyl mit 2 bis 6 Kohlenstoffatomen steht und
- X: für Halogen, Hydroxy, Amino, Mercapto steht,
- X: weiterhin für einen jeweils gegebenenfalls durch Halogen, Cyano, Hydroxy, Carboxy, C₁-C₄-Alkoxy, C₁-C₄-Alkyl-carbonyl oder C₁-C₄-Alkoxy-carbonyl substituierten Rest der Reihe Alkyl, Alkoxy, Alkylthio, Alkylamino, Alkanoylamino oder Alkylsulfonylamino mit jeweils bis zu 6 Kohlenstoffatomen in den Alkylgruppen steht,
- X: weiterhin für einen jeweils gegebenenfalls durch Halogen, Cyano, Carboxy, C₁-C₄-Alkyl oder C₁-C₄-Alkoxy-carbonyl substituierten Rest der Reihe Cycloalkyloxy, Cycloalkylthio, Cycloalkylamino, Cycloalkylalkoxy, Cycloalkyl-alkylthio oder Cycloalkyl-alkylamino mit jeweils 3 bis 6 Kohlenstoffatomen in den Cycloalkylgruppen und gegebenenfalls 1 bis 4 Kohlenstoffatomen in den Alkylgruppen steht, oder
- X: weiterhin für einen jeweils gegebenenfalls durch Halogen, Cyano, Nitro, Hydroxy, Amino, Carboxy, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituierten Rest der Reihe Aryloxy, Arylthio, Arylamino, Arylcarbonyl, Arylsulfonyl, Arylalkyl, Arylalkoxy,Arylalkylthio, Arylalkylamino, Arylalkylcarbonyl oder Arylalkylsulfonyl mit jeweils 6 oder 10 Kohlenstoffatomen in den Arylgruppen und gegebenenfalls 1 bis 4 Kohlenstoffatomen in den Alkylgruppen steht.
Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- m: für die Zahlen 0, 1, 2, 3 oder 4 steht,
- n: für die Zahlen 0, 1 oder 2 steht,
- R¹: für einen jeweils gegebenenfalls durch Fluor substituierten Rest der Reihe Methyl, Ethyl, n-oder i-Propyl, n-, i- oder s-Butyl, Propenyl, Butenyl, Propinyl oder Butinyl steht,
- R²: für einen jeweils gegebenenfalls durch Fluor substituierten Rest der Reihe Methyl, Ethyl, n-oder i-Propyl, n-, i- oder s-Butyl, Propenyl, Butenyl, Propinyl oder Butinyl steht oder zusammen mit R¹ für Ethan-1,2-diyl (Dimethylen), Propan-1,3-diyl (Trimethylen) oder Butan-1,4-diyl (Tetramethylen) steht und
- X: für Fluor, Chlor, Brom, Hydroxy, Amino, Mercapto steht,
- X: weiterhin für einen jeweils gegebenenfalls durch Fluor, Chlor, Brom, Cyano, Hydroxy, Carboxy, Methoxy, Ethoxy, Methoxycarbonyl oder Ethoxycarbonyl substituierten Rest der Reihe Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, s-, i- oder t-Butylthio, Methylamino, Ethylamino, n- oder i-Propylamino, n-, i-, s- oder t-Butylamino, Acetylamino, Propionylamino, Butyroylamino, Methylsulfonylamino, Ethylsulfonylamino, n- oder i-Propylsulfonylamino, n-, i-, s- oder t-Butylsulfonylamino steht,
- X: weiterhin für einen jeweils gegebenenfalls durch Fluor, Chlor, Brom, Cyano, Carboxy, Methyl, Ethyl, n- oder i-Propyl, Methoxycarbonyl oder Ethoxycarbonyl substituierten Rest der Reihe Cyclopentyloxy, Cyclohexyloxy, Cyclopentylthio, Cyclohexylthio, Cyclopentylamino, Cyclohexylamino, Cyclopentylmethoxy, Cyclohexylmethoxy, Cyclopentyl-methylthio, Cyclohexylmethylthio, Cyclopentylmethylamino oder Cyclohexyl-methylamino steht, oder
- X: weiterhin für einen jeweils gegebenenfalls durch Fluor, Chlor, Brom, Cyano, Nitro, Hydroxy, Amino, Carboxy, Methyl, Ethyl, Trifluormethyl, Methoxy, Ethoxy, Difluormethoxy, Trifluormethoxy, Methylthio, Ethylthio, Difluormethylthio, Trifluormethylthio, Methylsulfinyl, Ethylsulfinyl, Methylsulfonyl, Ethylsulfonyl, Trifluormethylsulfonyl, Methoxycarbonyl oder Ethoxycarbonyl substituierten Rest der Reihe Phenoxy, Phenylthio, Phenylamino, Phenylcarbonyl, Phenylsulfonyl, Benzyl, Benzyloxy, Benzylthio, Benzylamino, Phenylacetyl oder Benzylsulfonyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise Diethylcarbamidsäurechlorid und 3-(2-Chlor-4-cyano-5-fluor-phenylthio)-1H-1,2,4-triazol als Ausgangsstoffe, für eine anschließende Oxidationsreaktion Hydrogenperoxid als Oxidationsmittel und für eine nachfolgende Substitutionsreaktion Hydroxyessigsäuremethylester als Reaktionspartner, so kann der Reaktionsablauf beim erfindungsgemäßen Verfahren durch das folgende Formelschema skizziert werden: 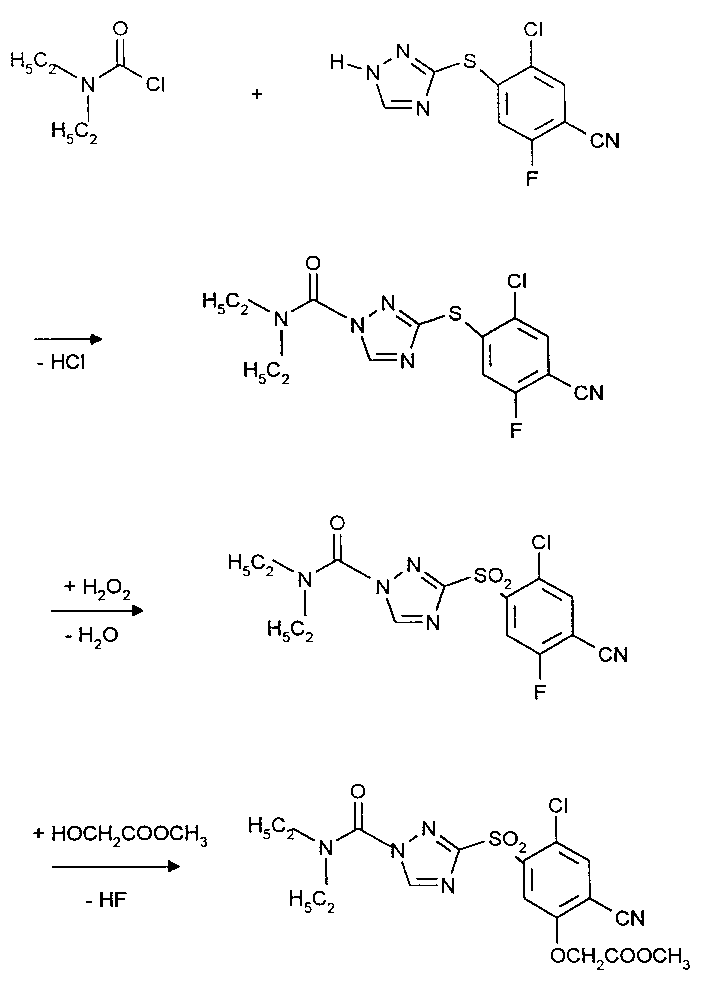
Die beim erfindungsgemäßen Verfahren zur Herstellung der Verbindungen der allgemeinen Formel (I) als Ausgangsstoffe zu verwendenden Carbamidsäurechloride sind durch die Formel (II) allgemein definiert. In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden.

Die Ausgangsstoffe der Formel (II) sind bekannte organische Synthesechemikalien.

Die beim erfindungsgemäßen Verfahren weiter als Ausgangsstoffe zu verwendenden substituierten Triazole sind durch die Formel (III) allgemein definiert. In der Formel (III) haben m, n und X vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für m, n und X angegeben wurden.

Die Ausgangsstoffe der Formel (III) sind noch nicht aus der Literatur bekannt; sie sind als neue Stoffe Gegenstand der vorliegenden Anmeldung.

Man erhält die neuen substituierten Triazole der allgemeinen Formel (III), wenn man das Mercaptotriazol der Formel (V) 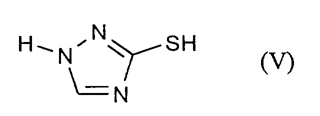
mit Cyanoarylverbindungen der allgemeinen Formel (VI) 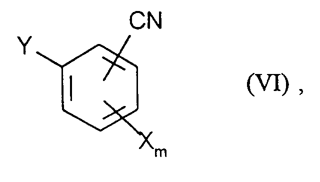
in welcher
- m und X: die oben angegebene Bedeutung haben und
- Y: für Halogen, vorzugsweise für Fluor oder Chlor steht,
gegebenenfalls in Gegenwart eines Säureakzeptors, wie z.B. Natriummethanolat, und gegebenenfalls in Gegenwart eines Verdünnungsmittels, wie z.B. Methanol, bei Temperaturen zwischen 0°C und 100°C umsetzt und nach üblichen Methoden aufarbeitet (vgl. die Herstellungsbeispiele).

Die Ausgangsstoffe der Formeln (V) und (VI) sind bekannte organischen Synthesechemikalien.

Das erfindungsgemäße Verfahren zur Herstellung der substituierten Carbamoyltriazole der Formel (I) wird vorzugsweise in Gegenwart eines Säureakzeptors durchgeführt. Als Säureakzeptoren kommen die üblichen anorganischen oder organischen Basen bzw. Säurebindemittel in Betracht. Hierzu gehören beispielsweise Alkalimetall- oder Erdalkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethyl-anilin, N,N-Dimethyl-benzylamin, Pyridin, Methylpyridine, N-Methylpiperidin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Auch von den oben genannten Stickstoffbasen können einige, wie z.B. Pyridin, im Überschuß eingesetzt als Verdünnungsmittel dienen.

Geeignete Oxidationsmittel für die gegebenenfalls als Folgestufe durchzuführende Oxidationsreaktion sind beispielsweise Sauerstoff, Ozon, Hydrogenperoxid, Chlor, Chlorlauge, Kaliumpermanganat, Perameisensäure, Peressigsäure, Perpropionsäure sowie gegebenenfalls halogenierte Perbenzoesäuren.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise bei Temperaturen zwischen 10°C und 80°C.
Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen wie Getreide, Rüben, Soja und Baumwolle sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfüron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate, Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

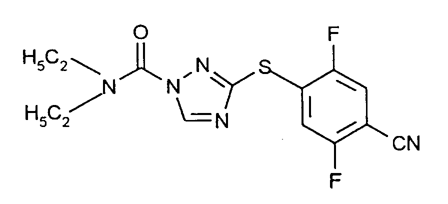

Eine Mischung aus 3,5 g (14,7 mMol) 3-(4-Cyano-2,5-difluor-phenylthio)-1H-1,2,4-triazol, 2,1 g (15,3 mMol) N,N-Diethyl-carbamidsäurechlorid und 20 ml Pyridin wird 3 Tage bei 20°C gerührt. Dann wird mit Wasser auf etwa das doppelte Volumen verdünnt, abfiltriert und nacheinander mit 1N-Salzsäure und mit Wasser gewaschen.

Man erhält 5,5 g (91,5% der Theorie) 3-(4-Cyano-2,5-difluor-phenylthio)-1-diethylaminocarbonyl-1H-1,2,4-triazol vom Schmelzpunkt 80°C.

### Beispiel 2

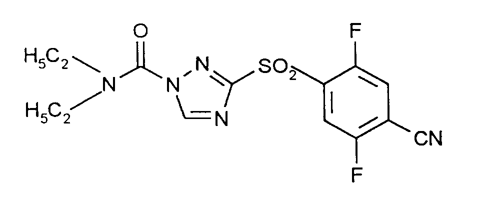

Eine Mischung aus 4,0 g (10 mMol) 3-(4-Cyano-2,5-difluor-phenylthio)-1-diethylaminocarbonyl-1H-1,2,4-triazol, 4,6 g (22 mMol) 3-Chlor-perbenzoesäure (80%ig) und 75 ml Chloroform wird zunächst bei 0°C 30 Minuten und dann bei 20°C 15 Stunden gerührt. Nach Zugabe von weiteren 0,9 g (4,5 mMol) 3-Chlor-perbenzoesäure wird das Gemisch weitere 15 Stunden bei 20°C gerührt. Dann wird dreimal mit gesättigter Natriumcarbonatlösung geschüttelt und die organische Phase im Vakuum eingeengt. Der Rückstand wird in Essigsäureethylester aufgenommen, das Produkt durch Zugabe von Hexan ausgefällt und durch Abfiltrieren isoliert.

Man erhält 1,2 g (32,5% der Theorie) 3-(4-Cyano-2,5-difluor-phenylsulfonyl)-1-diethylaminocarbonyl-1H-1,2,4-triazol vom Schmelzpunkt 98°C.

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

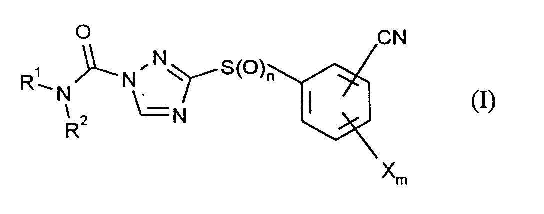

**Tabelle 1**

| Beispiele für die Verbindungen der Formel (I) | | | | | | | |
|---|---|---|---|---|---|---|---|
| Bsp.-Nr. | m | n | R¹ | R² | Position von CN | (Position-) X | Schmelzpunkt (°C) |
| 3 | 2 | 0 | CH₃ | CH₃ | 4 | (2,5-)F | 59 |
| 4 | 2 | 0 | C₂H₅ | C₂H₅ | 4 | (2-)Cl, (5-)F | |
| 5 | 2 | 1 | C₂H₅ | C₂H₅ | 4 | (2-)Cl, (5-)F | |
| 6 | 2 | 2 | C₂H₅ | C₂H₅ | 4 | (2-)Cl, (5-)F | |
| 7 | 0 | 0 | C₂H₅ | C₂H₅ | 4 | - | |
| 8 | 0 | 1 | C₂H₅ | C₂H₅ | 4 | - | |
| 9 | 0 | 2 | C₂H₅ | C₂H₅ | 4 | - | |
| 10 | 2 | 2 | CH₃ | CH₃ | 4 | (2,5-)SCH₃ | |
| 11 | 2 | 2 | CH₃ | CH₃ | 4 | (2,5-)OCH₃ | |
| 12 | 2 | 2 | C₂H₅ | C₂H₅ | 4 | (2-)Cl, (5-)NH₂ | |
| 13 | 2 | 2 | C₂H₅ | C₂H₅ | 4 | (2-)Cl, | |
| | | | | | | (5-)NHSO₂CH₃ | |
| 14 | 1 | 2 | C₂H₅ | C₂H₅ | 4 | (2-)F | |
| 15 | 1 | 2 | C₂H₅ | C₂H₅ | 4 | (2-)OCH₃ | |
| 16 | 1 | 2 | CH₃ | CH₃ | 4 | (2-)SC₂H₅ | |
| 17 | 1 | 2 | CH₃ | CH₃ | 4 | (3-)F | |
| 18 | 1 | 2 | C₂H₅ | C₂H₅ | 4 | (3-)F | |
| 19 | 1 | 2 | C₃H₇ | C₃H₇ | 4 | (3-)F | |
| 20 | 1 | 2 | C₂H₅ | C₂H₅ | 4 | (3-)NHSO₂C₂H₅ | |
| 21 | 1 | 2 | -(CH₂)₆- | | 4 | (3-)F | |
| 22 | 1 | 2 | -(CH₂)₅- | | 4 | (3-)F | |
| 23 | 1 | 2 | -(CH₂)₄- | | 4 | (3-)F | |
| 24 | 2 | 2 | -(CH₂)₅- | | 4 | (2,5-)F | |
| 25 | 2 | 0 | C₂H₅ | C₂H₅ | 4 | (2-)F, (5-)Cl | 107 |
| 26 | 2 | 1 | C₂H₅ | C₂H₅ | 4 | (2-)F, (5-)Cl | 140 |
| 27 | 2 | 2 | C₂H₅ | C₂H₅ | 4 | (2-)F, (5-)Cl | |
| 28 | 1 | 2 | C₂H₅ | CH₃ | 4 | 2-Cl | |
| 29 | 4 | 0 | C₂H₅ | C₂H₅ | 4 | (2,3,5,6-)F | 52 |
| 30 | 4 | 1 | C₂H₅ | C₂H₅ | 4 | (2,3,5,6-)F | |
| 31 | 4 | 2 | C₂H₅ | C₂H₅ | 4 | (2,3,5,6-)F | 32 |

### Ausgangsstoffe der Formel (III):Beispiel (III-I)

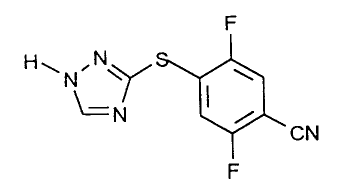

Eine Mischung aus 5,3 g (52 mMol) 3-Mercapto-1H-1,2,4-triazol, 8,2 g (52 mMol) 2,4,5-Trifluor-benzonitril, 2,8 g (52 mMol) Natriummethanolat und 50 ml Methanol wird 15 Stunden bei 20°C und dann weitere 12 Stunden bei 60°C sowie 8 Stunden unter Rückfluß gerührt. Nach Einengen auf etwa das halbe Volumen und Abkühlen wird das kristallin abgeschiedene Produkt durch Abfiltrieren isoliert.

Man erhält 4,0 g (32% der Theorie) 3-(4-Cyano-2,5-difluor-phenylthio)-1H-1,2,4-triazol vom Schmelzpunkt 185°C.

### Anwendungsbeispiele:

In den folgenden Anwendungsbeispielen wurde die nachstehend aufgeführte Verbindung (A) als Vergleichssubstanz eingesetzt: 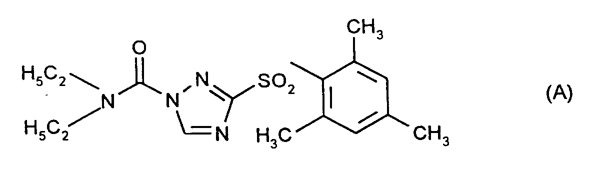
1-(Diethylaminocarbonyl)-3-(2,4,6-trimethylphenylsulfonyl)-1,2,4-triazol (bekannt aus EP-A 332 133).

### Beispiel A

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung wurde 1 Gewichtsteil Wirkstoff mit der angegebenen Menge Lösungsmittel vermischt, die angegebene, Menge Emulgator zugegeben und das Konzentrat anschließend mit Wasser auf die gewünschte Konzentration verdünnt.

Im Freiland wurden Parzellen mit Testpflanzen, welche eine Höhe von etwa 3 - 10 cm hatten, mit einer solchen Menge der Wirkstoffzubereitung bespritzt, daß eine gleichmäßige Benetzung der Pflanzen eintrat. Entscheidend dabei ist die Aufwandmenge des Wirkstoffes pro Flächeneinheit. Nach drei Wochen wurde der Schädigungsgrad der Pflanzen bonitiert in % Schädigung im Vergleich zur unbehandelten Kontrolle. Es bedeuten:
0 % = unbehandelte Kontrolle/keine Wirkung
100 % = totale Vernichtung.

In diesem Test zeigen beispielsweise die Verbindungen gemäß den Herstellungsbeispielen 1 und 2 bei einer Aufwandmenge von 1 000 g/ha bei sehr guter Verträglichkeit gegenüber Kulturpflanzen, wie z.B. Rüben und Baumwolle (0 %), starke Wirkung gegen Unkräuter wie z.B. Galium (80 %), Ipomoea (90 %), Alopecurus (50 %) und Cyperus (50 %).

### Beispiel B

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und der Boden nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
In diesem Test zeigen beispielsweise die Verbindungen gemäß den Herstellungsbeispielen 1 und 2 bei Aufwandmengen von 500 g/ha und und bei sehr guter Verträglichkeit gegenüber Kulturpflanzen, wie z.B. Gerste und Soja (0 %), starke Wirkung gegen Unkräuter wie Alopecerus (95 %), Lolium (80-95 %), Galinsoga (80-100 %), Abutilon (95 %) und Poa (100 %).

## Patentansprüche

1. Substituierte Carbamoyltriazole der allgemeinen Formel (I) 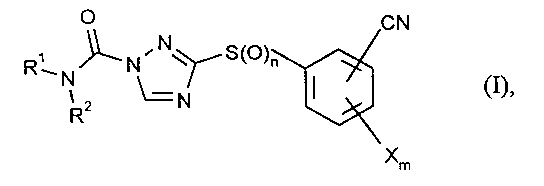 in welcher
m für die Zahlen 0 bis 4 steht,
n für die Zahlen 0, 1 oder 2 steht,
R¹ für einen jeweils gegebenenfalls substituierten Rest der Reihe Alkyl, Alkenyl oder Alkinyl steht,
R² für einen jeweils gegebenenfalls substituierten Rest der Reihe Alkyl, Alkenyl oder Alkinyl steht oder zusammen mit R¹ für Alkandiyl steht und
X für Halogen, Hydroxy, Amino, Mercapto oder für einen jeweils gegebenenfalls substituierten Rest der Reihe Alkyl, Alkoxy, Alkylthio, Alkylamino, Alkanoylamino, Alkylsulfonylamino, Cycloalkyloxy, Cycloalkylthio, Cycloalkylamino, Cycloalkylalkoxy, Cycloalkylalkylthio, Cycloalkylalkylamino, Aryloxy, Arylthio, Arylamino, Arylcarbonyl, Arylsulfonyl, Arylalkyl, Arylalkoxy, Arylalkylthio, Arylalkylamino, Arylalkylcarbonyl oder Arylalkylsulfonyl steht.

2. Substituierte Carbamoyltriazole der allgemeinen Formel (I) gemäß Anspruch 1, dadurch gekennzeichnet, daß
m für die Zahlen 0, 1, 2, 3 oder 4 steht,
n für die Zahlen 0, 1 oder 2 steht,
R¹ für einen jeweils gegebenenfalls durch Halogen substituierten Rest der Reihe Alkyl, Alkenyl oder Alkinyl mit jeweils bis zu 6 Kohlenstoffatomen steht,
R² für einen jeweils gegebenenfalls durch Halogen substituierten Rest der Reihe Alkyl, Alkenyl oder Alkinyl mit jeweils bis zu 6 Kohlenstoffatomen steht oder zusammen mit R¹ für Alkandiyl mit 2 bis 6 Kohlenstoffatomen steht und
X für Halogen, Hydroxy, Amino, Mercapto steht,
X weiterhin für einen jeweils gegebenenfalls durch Halogen, Cyano, Hydroxy, Carboxy, C₁-C₄-Alkoxy, C₁-C₄-Alkyl-carbonyl oder C₁-C₄-Alkoxy-carbonyl substituierten Rest der Reihe Alkyl, Alkoxy, Alkylthio, Alkylamino, Alkanoylamino oder Alkylsulfonylamino mit jeweils bis zu 6 Kohlenstoffatomen in den Alkylgruppen steht,
X weiterhin für einen jeweils gegebenenfalls durch Halogen, Cyano, Carboxy, C₁-C₄-Alkyl oder C₁-C₄-Alkoxy-carbonyl substituierten Rest der Reihe Cycloalkyloxy, Cycloalkylthio, Cycloalkylamino, Cycloalkylalkoxy, Cycloalkyl-alkylthio oder Cycloalkyl-alkylamino mit jeweils 3 bis 6 Kohlenstoffatomen in den Cycloalkylgruppen und gegebenenfalls 1 bis 4 Kohlenstoffatomen in den Alkylgruppen steht, oder
X weiterhin für einen jeweils gegebenenfalls durch Halogen, Cyano, Nitro, Hydroxy, Amino, Carboxy, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituierten Rest der Reihe Aryloxy, Arylthio, Arylamino, Arylcarbonyl, Arylsulfonyl, Aryl alkyl, Arylalkoxy, Arylalkylthio, Arylalkylamino, Arylalkylcarbonyl oder Arylalkylsulfonyl mit jeweils 6 oder 10 Kohlenstoffatomen in den Arylgruppen und gegebenenfalls 1 bis 4 Kohlenstoffatomen in den Alkylgruppen steht.

3. Substituierte Carbamoyltriazole der allgemeinen Formel (I) gemäß Anspruch 1, dadurch gekennzeichnet, daß
m für die Zahlen 0, 1, 2, 3 oder 4 steht,
n für die Zahlen 0, 1 oder 2 steht,
R¹ für einen jeweils gegebenenfalls durch Fluor substituierten Rest der Reihe Methyl, Ethyl, n-oder i-Propyl, n-, i- oder s-Butyl, Propenyl, Butenyl, Propinyl oder Butinyl steht,
R² für einen jeweils gegebenenfalls durch Fluor substituierten Rest der Reihe Methyl, Ethyl, n-oder i-Propyl, n-, i- oder s-Butyl, Propenyl, Butenyl, Propinyl oder Butinyl steht oder zusammen mit R¹ für Ethan-1,2-diyl (Dimethylen), Propan-1,3-diyl (Trimethylen) oder Butan-1,4-diyl (Tetramethylen) steht und
X für Fluor, Chlor, Brom, Hydroxy, Amino, Mercapto steht,
X weiterhin für einen jeweils gegebenenfalls durch Fluor, Chlor, Brom, Cyano, Hydroxy, Carboxy, Methoxy, Ethoxy, Methoxycarbonyl oder Ethoxycarbonyl substituierten Rest der Reihe Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, s-, i- oder t-Butylthio, Methylamino, Ethylamino, n- oder i-Propylamino, n-, i-, s- oder t-Butylamino, Acetylamino, Propionylamino, Butyroylamino, Methylsulfonylamino, Ethylsulfonylamino, n- oder i-Propylsulfonylamino, n-, i-, s- oder t-Butylsulfonylamino steht,
X weiterhin für einen jeweils gegebenenfalls durch Fluor, Chlor, Brom, Cyano, Carboxy, Methyl, Ethyl, n- oder i-Propyl, Methoxycarbonyl oder Ethoxy-carbonyl substituierten Rest der Reihe Cyclopentyloxy, Cyclohexyloxy, Cyclopentylthio, Cyclohexylthio, Cyclopentylamino, Cyclohexylamino, Cyclopentylmethoxy, Cyclohexylmethoxy, Cyclopentyl-methylthio, Cyclohexylmethylthio, Cyclopentylmethylamino oder Cyclohexyl-methylamino steht, oder
X weiterhin für einen jeweils gegebenenfalls durch Fluor, Chlor, Brom, Cyano, Nitro, Hydroxy, Amino, Carboxy, Methyl, Ethyl, Trifluormethyl, Methoxy, Ethoxy, Difluormethoxy, Trifluormethoxy, Methylthio, Ethylthio, Difluormethylthio, Trifluormethylthio, Methylsulfinyl, Ethylsulfinyl, Methylsulfonyl, Ethylsulfonyl, Trifluormethylsulfonyl, Methoxycarbonyl oder Ethoxycarbonyl substituierten Rest der Reihe Phenoxy, Phenylthio, Phenylamino, Phenylcarbonyl, Phenylsulfonyl, Benzyl, Benzyloxy, Benzylthio, Benzylamino, Phenylacetyl oder Benzylsulfonyl steht.

4. Verfahren zur Herstellung substituierter Carbamoyltriazole der allgemeinen Formel (I) gemäß Anspruch 1 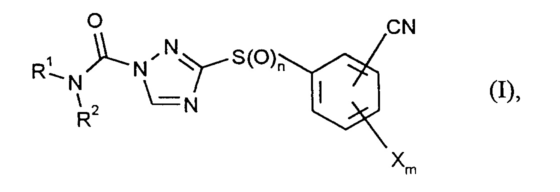 in welcher R¹, R², X, m und n die in Anspruch 1 genannten Bedeutungen haben, dadurch gekennzeichnet, daß man Carbamidsäurechloride der allgemeinen Formel (II) 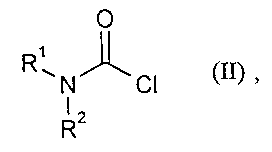 in welcher
R¹ und R²die in Anspruch 1 genannten Bedeutungen haben
mit substituierten Triazolen der allgemeinen Formel (III) 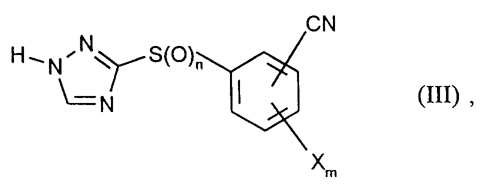 in welcher
m, n und X die in Anspruch 1 genannten Bedeutungen haben,
gegebenenfalls in Gegenwart eines Säureakzeptors und gegebenenfalls in Gegenwart eines Verdünnungsmittels umsetzt,
gegebenenfalls die so erhaltenen Verbindungen der Formel (I), in welcher n für 0 steht, durch Umsetzung mit einem Oxidationsmittel in entsprechende Verbindungen der Formel (I), in welcher n für 1 oder 2 steht umwandelt und/oder gegebenenfalls die so erhaltenen Verbindungen der Formel (I), in welcher X für Halogen steht, durch Umsetzung mit Verbindungen der Formel (IV)
MX¹ (IV),
in welcher
M für Wasserstoff oder ein Metalläquivalent steht und
X¹ mit Ausnahme von Halogen und Alkyl die oben für X angegebene Bedeutung hat,
in entsprechend derivatisierte Verbindungen der Formel (I) umwandelt.

5. Verfahren zur Bekämpfung von unerwünschten Pflanzen, dadurch gekennzeichnet, daß man substituierte Carbamoyltriazole der Formel (I) gemäß den Ansprüchen 1 bis 4 auf unerwünschte Pflanzen und/oder ihren Lebensraum einwirken läßt.

6. Verwendung von substituierten Carbamoyltriazolen der Formel (I) gemäß den Ansprüchen 1 bis 4 zur Bekämpfung von unerwünschten Pflanzen.

7. Verfahren zur Herstellung von herbiziden Mitteln, dadurch gekennzeichnet, daß man substituierte Carbamoyltriazole der Formel (I) gemäß den Ansprüchen 1 bis 4 mit Streckmitteln und/oder oberflächenaktiven Substanzen vermischt.

8. Herbizide Mittel, gekennzeichnet durch einen Gehalt an substituierten Carbamoyltriazolen der Formel (I) gemäß den Ansprüchen 1 bis 4.

9. Substituierte Triazole der allgemeinen Formel (III) 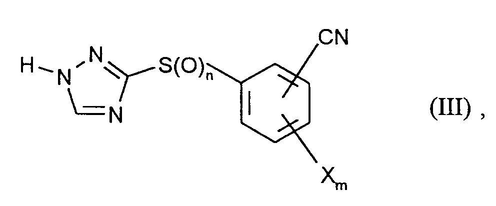 in welcher
m für die Zahlen 0 bis 4 steht,
n für die Zahlen 0, 1 oder 2 steht,
X für Halogen, Hydroxy, Amino, Mercapto oder für einen jeweils gegebenenfalls substituierten Rest der Reihe Alkyl, Alkoxy, Alkylthio, Alkylamino, Alkanoylamino, Alkylsulfonylamino, Cycloalkyloxy, Cycloalkylthio, Cycloalkylamino, Cycloalkylalkoxy, Cycloalkylalkylthio, Cycloalkylalkylamino, Aryloxy, Arylthio, Arylamino, Arylcarbonyl, Arylsulfonyl, Arylalkyl, Arylalkoxy, Arylalkylthio, Arylalkylamino, Arylalkylcarbonyl oder Arylalkylsulfonyl steht.

10. Verfahren zur Herstellung der substituierten Triazole der Formel (III) gemäß Anspruch 9 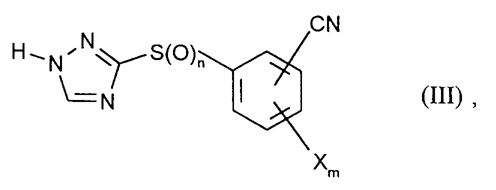 in welcher X, m und n die in Anspruch 9 genannten Bedeutungen haben, dadurch gekennzeichnet, daß man Mercaptotriazol der Formel (V) 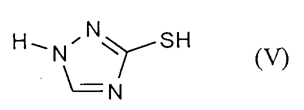 mit Cyanoarylverbindungen der allgemeinen Formel (VI) 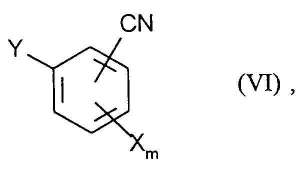 in welcher
m und X die in Anspruch 9 angegebene Bedeutung haben und
Y für Halogen steht,
gegebenenfalls in Gegenwart eines Säureakzeptors und gegebenenfalls in Gegenwart eines Verdünnungsmittels bei Temperaturen zwischen 0°C und 100°C umsetzt und nach üblichen Methoden aufarbeitet.
